(19) Europäisches Patentamt
European Patent Office
Office européen des brevets

(11) **EP 4 557 183 A1**

(12) **EUROPEAN PATENT APPLICATION**

(43) Date of publication:
**21.05.2025 Bulletin 2025/21**

(21) Application number: **23209774.1**

(22) Date of filing: **14.11.2023**

(51) International Patent Classification (IPC):
*G06N 20/00* (2019.01)

(52) Cooperative Patent Classification (CPC):
**G06N 20/00;** G06N 3/045; G06N 3/098

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Siemens Healthineers AG
91301 Forchheim (DE)**

(72) Inventors:
• **JEREBKO, Anna
Paoli, 19301 (US)**
• **KOLLERATHU, Varghese
560066 Bengalurur, Karnataka (IN)**

(74) Representative: **Kraus & Lederer PartGmbB et al
Thomas-Wimmer-Ring 15
80539 München (DE)**

(54) **CLIENT-BASED RE-TRAINING AND EVALUATION OF ARTIFICIAL-INTELLIGENCE MODEL**

(57) The present disclosure relates deployment of an artificial intelligence model from a central server (91). The disclosure further relates to client-based re-training and validation of an artificial intelligence model at multiple clients (92, 93, 94).

FIG 8

## Description

TECHNICAL FIELD

Various examples of the disclosure generally pertain to retraining an artificial-intelligence model at multiple clients and evaluating the retrained artificial-intelligence model at the multiple clients.

BACKGROUND

Artificial intelligence (AI) models (sometimes also referred to as machine learning models) are functions that are trained using pairs of input and output data forming a training dataset. Various types of AI models are known, including support vector machines and deep neural networks.

The parameters of the AI models are set in an optimization, to best reproduce the output data based on the input data.

Training of an AI model is typically computationally expensive. Furthermore, the accuracy of the AI model depends on the training datasets. For example, it is desirable that the training dataset comprehensively samples the input space observed in practical deployment scenarios. Otherwise, the predictions made by the AI model can be inaccurate.

To address such aspects, techniques are known to, firstly, pre-train an AI model at a central authority based on an initial training dataset. The initial training dataset is typically collected by experts. Individual input-output data is curated by experts.

Then, the pre-trained AI model can be deployed to multiple clients. The clients are devices or nodes that are not under direct influence of the central authority. I.e., they can independently use the pre-trained AI model. The clients can perform inference based on the pre-trained AI model. Performing inference means that input data is collected and predictions of the AI model are made, without ground truth being available.

Scenarios are known in which users then confirm or discard predictions made by the AI model, thereby generating ground-truth output data. The pairs of input-output data thus can be included in a further training dataset that is generated at each client. Based on such training datasets that are determined at the clients, it is possible to re-train the AI model.

Such re-training of the AI model may be executed at the clients, in a decentralized manner without involving the central authority. I.e., different clients collect different training datasets and locally re-train the AI model to obtain a respective updated training state of the AI model. This has the advantage of not having to provide the training datasets to the central authority. Privacy and data security can thereby be ensured.

However, such techniques of decentralized re-training of AI model face certain restrictions and drawbacks. In a real-world setting, the input-output data residing at participating clients is noisy in nature. Some clients may produce bad training datasets. Due to this, the performance of the re-trained AI models could potentially deteriorate.

SUMMARY

Accordingly, a need exists for re-training AI models based on training datasets acquired at multiple clients. A need exists for techniques that mitigate or overcome at least some of the above-identified restrictions and drawbacks.

This need is met by the features of the independent claims. The features of the dependent claims define embodiments.

Various disclosed aspects pertain to acquisition of training datasets for re-training an AI model at multiple clients. Various aspects pertain to re-training of the AI model at the multiple clients. Various disclosed aspects pertain to an iterative and decentralized approach to determine clients that generate noisy / bad-quality training datasets. Various aspects pertain to evaluation of the performance of the AI model in a training state after re-training. Various aspects pertain to cross-evaluation of the performance at the multiple clients.

The present disclosure provides a computer-implemented method. The method includes deploying an artificial intelligence model. The artificial intelligence model is deployed in a first training state. The artificial intelligence model is deployed to multiple clients. The method also includes, at each of the multiple clients: acquiring a respective training dataset. The method further includes, at each of the multiple clients: re-training the artificial intelligence model; this re-training is done to obtain the artificial intelligence model in a respective second training state. The method also includes associating the multiple clients with multiple groups. The method also includes for each of the multiple groups: aggregating weights of the artificial intelligence model in the second training state associated with clients within the respective group, to obtain the artificial intelligence model in a respective third training state. The method further includes evaluating the artificial intelligence model in each of the third training states.

Further, the present disclosure provides a computer-implemented method for use in a client, the method comprising: obtaining, from a central authority (e.g., a central server), an artificial intelligence model in a first training state; performing inference based on the artificial intelligence model in the first training state; acquiring a training dataset based on said performing of the inference; re-training the artificial intelligence model based on the training dataset, to obtain the artificial intelligence model in a second training state; providing the artificial intelligence model in the second training state to at least one of the central authority and one or more further clients; establishing the artificial intelligence model in multiple third training states based on information obtained from at least one of the central authority or the one or more further clients; evaluating the artificial intelligence model in each of the multiple third training states based on benchmarking against performance of the artificial intelligence model in the third training state and based on the training dataset.

Further, the present disclosure provides a computer-implemented method for use in a central authority, the method comprising: deploying an artificial intelligence model in a first training state to multiple clients; obtaining the artificial intelligence model in respective second training states from each of the multiple clients; associating the multiple clients with multiple groups; for each of the multiple groups: aggregating weights of the artificial intelligence model in the second training states associated with clients within the respective group, to obtain the artificial intelligence model in a respective third training state; and providing the artificial intelligence model in the third training states to the multiple clients for evaluation.

It is to be understood that the features mentioned above and those yet to be explained below may be used not only in the respective combinations indicated, but also in other combinations or in isolation without departing from the scope of the invention.

BRIEF DESCRIPTION OF THE DRAWINGS

FIG. 1 schematically illustrates a system of a central server implementing a central authority for multiple clients of the system according to various examples.

FIG. 2 schematically illustrates a device according to various examples, wherein the device may implement a central authority or a client.

FIG. 3 schematically illustrates functional aspects of a client according to various examples.

FIG. 4 schematically illustrates functional aspects of grouping of multiple clients according to various examples.

FIG. 5 is a flowchart of a method according to various examples.

FIG. 6 is a flowchart of a method according to various examples.

FIG. 7 is a flowchart of a method according to various examples.

FIG. 8 is a flowchart of a method according to various examples.

DETAILED DESCRIPTION

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

Various examples of the disclosure generally pertain to prediction using AI models. As a general rule, various types of AI models can be subject to the techniques disclosed herein, e.g., support vector machines, deep neural networks, random forest algorithms, to give just a few examples.

The prediction using AI models can be used in various use cases. For example, AI models can operate based on medical data such as medical imaging data. AI models can assess one or more properties of the medical imaging data. For instance, it would be possible to perform image reconstruction based on magnetic-resonance imaging (MRI) data. For example, it would be possible to detect one or more features in medical imaging data, e.g., lesions in MRI images, fractures in computed tomography (CT) images, to give just a few examples. Anatomical structures can be segmented in images. Treatment planning based on AI models is another use case.

Various examples of the disclosure generally pertain to training processes for training the AI models. An iterative training process is disclosed. The AI model is initially present in an initial training state and a re-trained, to obtain an updated training state. This can be repeated multiple times.

For example, training a deep neural network (as an example of an AI model) includes using a training dataset composed of pairs of input-output data. Each pair provides an example from which the deep neural network can learn by adjusting its internal parameters to map inputs to expected outputs, known as the ground truth. The objective during training is optimization of the network's parameters to minimize the discrepancy between the network's predictions and the ground truth. This optimization is typically achieved using the gradient descent algorithm. For each input-output pair, the network's current prediction is compared to the ground truth to compute an error. Backpropagation is then employed to calculate the gradients of this error with respect to each network parameter. These gradients indicate how much each parameter should be adjusted to reduce the overall error. The gradient descent algorithm uses these gradients to update the network's parameters in a direction that decreases the error. This process is iteratively performed on the entire training dataset until the network's predictions align closely with the ground truth for a majority of input-output pairs.

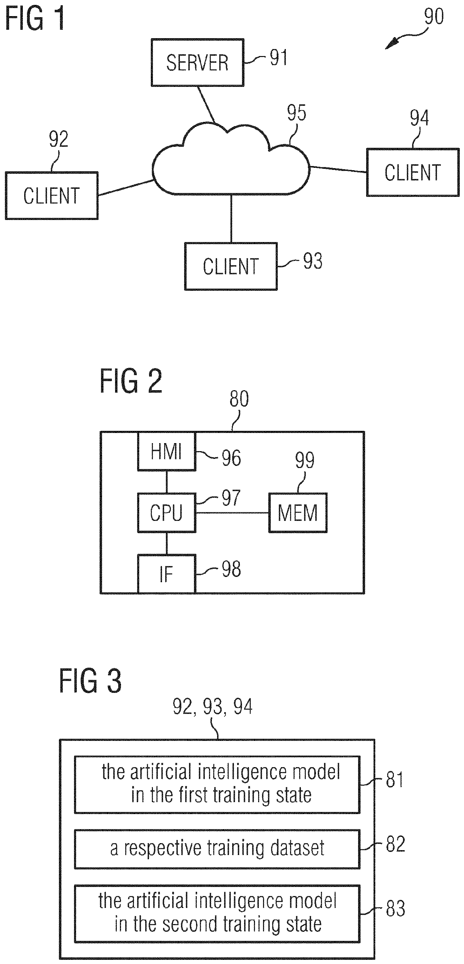

According to various example, the re-training and specifically the computationally-expensive optimization is executed at multiple clients, based on training datasets that are locally acquired at the multiple clients. This may be referred to as distributed re-training. A central authority is not required to evaluate the training datasets prior to executing the re-training. This preserves privacy, because the training dataset does not need to be exposed to the central authority or other clients. By implementing the retraining in a distributed manner at multiple clients, the training datasets can be retained at each of the multiple clients. It is not required to share the training datasets amongst the multiple clients or with a central authority. Thereby, privacy is maintained. A respective system is disclosed in FIG. 1.

FIG. 1 schematically illustrates a system 90. The system 90 includes a central server 91, implementing a central authority of the system 90. For instance, the central server 91 could be maintained and operated by a developer of an AI model. The developer of the AI model may also be a manufacturer of certain medical imaging equipment operated at sites of multiple clients 92, 93, 94.

As a general rule, depending on the particular use case, clients can be implemented differently. For instance, for processing of medical image data, clients can be implemented by computers at a hospital, connected to radiology equipment, e.g., connected to a magnetic resonance tomography (MRT) apparatus or to a CT apparatus.

The clients 92, 93, 94 as depicted in FIG. 1 are connected via the Internet 95 with the central server 91. The central server 91 can provide certain data or information to each of the clients 92, 93, 94. For instance, the central server 91 can deploy an AI model in an initial training state to each of the clients 92, 93, 94; thereby, facilitating inference tasks at each of the clients 92, 93, 94. The clients 92, 93, 94 can also provide data to the central server, e.g., a re-trained AI model (in a further training state). Typically, input data used for inference by the AI model is locally retained at each of the clients 92, 93, 94, i.e., not shared with the central server 91 or other clients.

FIG. 2 schematically illustrates a device 80. The device 80 can implement any one of the central server 91 or the clients 92, 93, 94 of the system 90 of FIG. 1. The device 80 includes a processor 97 coupled to a memory 99. The processor 97 can load program code from the memory 99 and execute the program code. Based on execution of the program code, the processor 97 can perform one or more tasks as disclosed herein, e.g.: deploying an AI model to multiple clients; obtaining an AI model from a central authority; communicating with one or more clients and/or a central authority; controlling a graphical user interface output to a user via a human machine interface 96; obtaining medical imaging data via a communication interface 98; processing the medical imaging data based on an AI model; re-training the AI model, to obtain the AI model in an updated training state; communicating weights of the AI model via the communication interface 98; etc.

FIG. 3 schematically illustrates re-training an AI model at each of the clients 92, 93, 94. The respective client 92, 93, 94 stores data defining the AI model in a first training state 81. The respective client 92, 93, 94 also stores a training dataset 82. Based on the training dataset 82, a retraining of the AI model in the first training state 81 can be performed, to obtain data defining the AI model in a second training state 83.

The training dataset 82 can be locally acquired. I.e., the training dataset can include pairs of input-output data acquired at one or more sensors or machines or processes executed locally at the respective client 92, 93, 94, e.g., at one or more machines connected with the respective client 92, 93, 94 via a local area network (rather than via a public network such as the Internet 95). The training dataset 82 can be acquired at one or machines that are under control of the same operator that also operates the respective client 92, 93, 94. Typically, the training dataset 82 is of limited size, e.g., if compared to a training dataset used in the initial training of the AI model at a central authority such as the central server 91 (e.g., acquired using a dedicated measurement campaign).

Various techniques are based on the finding that re-training on a small training dataset arising from a single client may yield a new respective training state of the AI model that does not generalize well on other clients. In the case of data poisoning i.e., incorrect labeling of data or poor labeling occurs at a client, an AI model trained on such client(s) may lead to poor generalization or sub-optimal performance on other clients.

Due to the reason above, it is desirable evaluate the performance of the AI models in the second training state 83. In one example, such evaluation can include a cross-evaluation process at the multiple clients.

In a reference implementation of such cross-evaluation process, if there are $N$ clients re-training the AI model in the first training state, then there are $N$ second training states of the AI model available after re-training. This would mean there are $N$ instances of the AI models that need to be evaluated and they need to be communicated to all the available clients. From the perceptive of a client, not only should it train a model on its local data, but it needs to evaluate $N$ training states (its own training state and the training states from remaining $N-1$ clients) on its training dataset. This reference implementation of the cross-evaluation process, apart from being time consuming, may not viable as the resource requirement needed is prohibitively large.

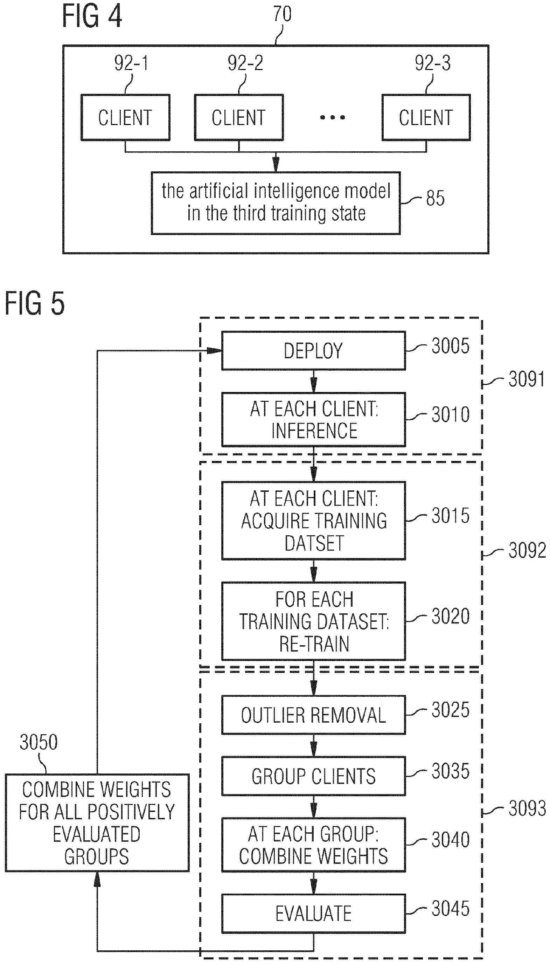

Accordingly, according to the disclosure, a concept of grouping multiple clients into groups for the purpose of evaluation of the new training states of the AI model is used. This is shown in FIG. 4.

FIG. 4 schematically illustrates aspects with respect to grouping multiple clients, here clients 92-1, 92-2, 92-3 that are part of a group 70. Illustrated in FIG. 4 is a scenario in which the AI model in multiple second training state 83-1, 83-2, 83-3 (as obtained at multiple clients 92-1, 92-2, 92-3) is combined, to obtain the AI model in a third training state 85.

More generally, it is possible associate the multiple clients with multiple groups. For example, all $N$ clients are divided into $M$ groups, and $M << N$. These groups may be non-overlapping or in other words mutually exclusive to each other. In the context of the example provided above, $N$ clients are broken down to groups (*Group1, Group2, Group3, ...., GroupM-1, GroupM*) and each group consists of $L$ clients, wherein

$$L= \frac{N}{M}.$$

Then, as shown in FIG. 4, the weights of the AI model in the second training states (i.e., as re-trained at each client) are aggregated within a group to form obtain the AI model in the third training state. In other words, the weights of the L individual re-trained AI models are fused to form a single training state.

As a general rule, in the various disclosed scenarios, such combination / fusing of the weights can use various aggregation strategies, e.g., averaging of the weights or a weighted average of the weights. The number of datapoints that used to train the respective training state being fused could be used as weighing scheme. I.e., the larger the underlying training dataset, the larger the weight.

By combining multiple training states, the number of training states to be evaluated can be reduced from N to M namely *Model 1, Model 2, Model 3,... Model M.*

It is then possible to evaluate the instances of the AI model in the third training states.

For instance, each third training state may be evaluated at the central authority. For this, the third training states can be communicated to a central server implementing the central authority. A benchmark dataset may be available at the central authority and used for said evaluation.

In other examples, a cross-evaluation process at the multiple clients may be used. For a cross-evaluation process, the AI model in each third training state is transferred to clients that form the remaining other groups. For instance, the third training state obtained for Group 1 is transferred to at least one client of each of Group2, Group3,..., GroupM and evaluated there, i.e., a cross validation is achieved. For example, the AI model in the third training state can be benchmarked against the AI model in the first training state, as previously deployed by the central authority. Such benchmarking can include executing inference based on the input data of the respective training dataset acquired at the client (or another dataset of pairs of input-output suitable for validation) and comparing a deviation from the ground truth output data of the training dataset. For larger deviations, poor performance is determined.

Then, two situations are conceivable: Firstly, it is possible that the AI model in the third training state performs better than the AI model in the first training state. I.e., the AI model in the third training state generalizes well on other clients and hence can be considered as replacement or an update to the current version of the AI model. A new deployment can be triggered. Secondly, it is also possible that the AI model in the third training state performs worse than the AI model in the first training state. The AI model in the third training state is not generalize well on other clients.

In such a scenario, it would be possible to break down the particular group of clients associated with the underperforming third training state into smaller subgroups, e.g., break down Group1 into B smaller subgroups (*SubGroup1_1, Subgroup1_2, SubGroup1_3, Subgroup1_4, ..., SubGroup1_B*), and $B << M$. I.e., responsive to evaluating of the AI model in the respective third training state not meeting a predefined benchmark, those clients previously associated with the respective group are re-associated with multiple newly formed (sub-)groups that replace the initial group. Each of the newly formed groups consists of P clients, wherein

$$P=\frac{M}{B}.$$ Then, the previously described steps can be reiterated for the newly formed subgroups. I.e., from each of the newly formed subgroup, the respective second training states of the AI model associated with the clients in that subgroup are combined to form a respective third training state. It is then possible to re-perform the cross-evaluation process. I.e., the third training states are then transferred to clients of the remaining groups and their performance is compared against the AI model in the first training state based on the respective local training dataset. If the performance of the newly determined third training state is non-inferior to that of the first training state, then they are retained; if the performance is inferior then the process mentioned above repeats.

The technique of starting with relatively large groups and break down those larger groups associated with underperforming third training states into smaller groups can be labeled as an "iterative cross-evaluation process". The clients are initially associated with a relatively large groups; this initially minimizes the computational efforts for the execution of the cross-evaluation process. However, if a poor performing third training state of the AI model is detected (associated with a relatively large group), then this group can be broken down into smaller groups in the process can be repeated. This helps to identify those clients that poison the data quality, e.g., providing poor quality training datasets.

Finally, all third training states passing the evaluation can be combined. I.e., the weights of all third training states passing the cross-evaluation process can be combined, e.g., by averaging or by determining a weighted average. Similar techniques as described above in connection with combining the weights of the AI model in the second training states to obtain the AI model in a respective third training state can also be applied. This yields a fourth training state that can then be re-deployed, e.g., by the central authority.

Since all constituents of the AI model in the fourth training have been successfully evaluated, it provides for a curated performance. Apart from enabling faster evaluation this approach also aids in determining outlier clients wherein there is a possibility for data poisoning and avoids them from contributing when it comes to creating the updated version of the algorithm. This information can also be taken into consideration to inform the participating client about potential data poisoning or even removing the client from the training step.

In a scenario wherein there are $N$ clients, then the number of evaluations that needs to carried out using conventional evaluation technique is equal to $N^2$. However, in the proposed method, the $N$ clients are divided into $M$ subgroups to yield $M$ AI models which need to be evaluated. Say, one of the $M$ models in the third training state underperforms, hence these clients is broken down further down further to form $B$ groups with a respective number of third training states which also need to be evaluated at $(M-1)$ subgroups. Therefore, the number of evaluations required in this scenario is equal to $[M^2 + (M-1) \times B]$. It should be noted here that $B<<M<<N$.

FIG. 5 is a flowchart of a method according to various examples. The method of FIG. 5 generally pertains to a client-based re-training of an AI model. For instance, the method of FIG. 5 can be executed by a system including a central server and multiple clients, e.g., by the system 90 of FIG. 1. Distributed processing at multiple devices is possible. For instance, processors at multiple devices can load program code from respective memories and execute the program code, to participate to the method of FIG. 5 (cf. FIG. 2: device 80).

At box 3005, an AI model in a first training state is deployed.

Deploying an AI model can include transmitting, by a central server (cf. FIG. 1) one or more messages to multiple clients (cf. FIG. 1: clients 92, 93, 94), e.g., via the Internet. Such messages can include the weights of the AI model in the first training state.

Deploying the AI model can include receiving one or more such messages at each of the multiple clients.

At box 3010, it is then possible to perform inference based on the AI model in the first training status deployed in box 3005. This means that input data is provided to the AI model in the first training state and output data is obtained from the AI model in the first training state after respective calculation. Ground truth needs not to be available when performing inference at 3010.

Accordingly, the grouping box 3091 corresponds to an inference phase for performing inference using the AI model.

Then, a re-training commences at grouping box 3092.

The re-training includes, at box 3015, acquiring training datasets, wherein a respective training dataset is acquired at each client. Thus, each training dataset is client-specific. Different clients have different training datasets. Each training dataset includes a collection of pairs of input data - output data. The output data is ground truth associated with a nominal prediction of the AI model. There are various options available for acquiring such training datasets and generally, acquiring the training dataset at box 3015 can be based on box 3010, i.e., based on performing of the inference. In particular, oftentimes it is possible that while at the time of performing inference the ground truth is not available, based on user interaction the output data is then positively validated as corresponding to the nominal output or altered by the user to constitute the ground truth. To give a concrete example: it would be possible that at box 3010 the AI model is used to segment a certain structure in medical imaging data, e.g., a lesion. Then, this segmentation prediction provided by the AI model in the first training state can be output to the user via a graphical user interface. The user may either simply confirm the segmentation as being correct, thereby generating the ground truth. Alternatively, the user may alter the segmentation, e.g., by locally adapting the segmentation map etc., thereby generating the ground truth. Over the course of time, thereby a training dataset of sufficient size for executing a retraining of the AI model is acquired at box 3015.

Eventually, it is then possible to retrain the AI model to obtain a second training state, at box 3020. This is executed at each of the multiple clients based on the local training dataset. Thereby, once completing box 3020, the number of second training states corresponds to the number of clients.

According to examples, it is possible that certain clients are preidentified as unreliable, e.g., by the central authority or based on local processes implemented at the clients. For instance, due to noisy measurement data etc., certain clients may be identified as outlier clients with a possibility for data poisoning. It is then generally possible to suppress an impact of the AI model in the respective second training state onto further processing, more specifically on a subsequently deployed training state of the AI model. There are different options for achieving such suppression of the impact of the respective client onto the formation of a further training state to be deployed and one option is illustrated in FIG. 5. Here, at box 3025, the respective second training state of the AI model is discarded for all clients that are labeled as outlier. For instance, this can be done by the central authority. It would also be possible to pre-configure such clients so that they are not required to re-training the AI model to even obtain the second training state.

At box 3035, all clients are associated with multiple groups. Each client may be associated with exactly a single group. The groups can be non-overlapping. It would also be possible that the groups are overlapping. The groups are logical collections of multiple clients. The groups are formed for the purpose of evaluating the AI model.

Accordingly, at box 3040, the weights of the AI model in the second training states associated with clients of any given group are respectively aggregated, to obtain the AI model in a respective third training state. Thus, at box 3040, a number of third training states is obtained that corresponds to the number of groups.

It is then possible at box 3045, to evaluate the AI model in each of the third training states, e.g., using a cross-evaluation process at the multiple clients. Thus, as will be appreciated from the above, boxes 3025, 3035, 3040, and 3045 correspond to an evaluation/validation, labeled by grouping box 3039.

Upon evaluating the AI model in each of the third training states at box 3045, weights of the AI model in those third training states that are associated with a positive result of said evaluating are aggregated at box 3050, to thereby obtain the AI model in a fourth training state.

Then, box 3005 can be re-executed, thereby deploying the AI model in the fourth training state. For this purpose, the AI model in the fourth training state can be communicated to a central authority or the aggregation of box 3050 can be directly implemented at the central authority.

As will be appreciated from the above, the computational complexity of the evaluation at box 3045 can be reduced by combining multiple respective second training states of the AI model into a corresponding third training state of the AI model at box 3040. The grouping at box 3035 can take into account multiple criteria such as the overall count of groups, number of clients per groups, and/or a size of the training dataset at each of the multiple clients.

For instance, it can be desirable that the number of pairs of input data - output data underlying each of the third training states is comparable across the groups. Thus, the groups can be formed such that the sum of sizes or the training datasets of all clients in each group is approximately stable across all groups. Also, the number of groups can be said to exceed a certain minimum threshold, e.g., four groups or ten groups, etc. This can also be based on the number of participating clients.

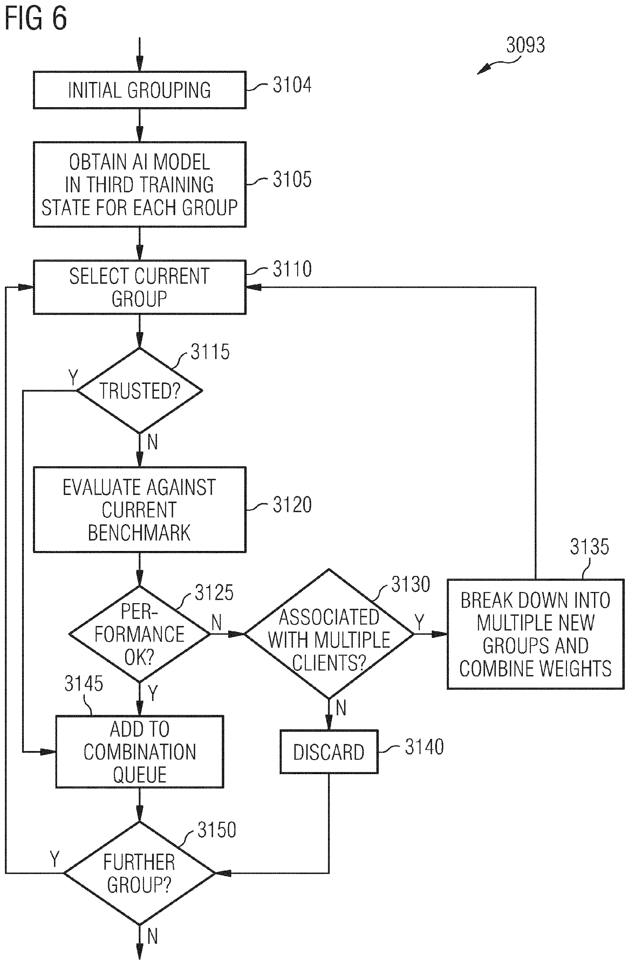

FIG. 6 is a flowchart of a method according to various examples. FIG. 6 illustrates a specific example implementation with respect to the evaluation process of grouping box 3093 of FIG. 5. The example implementation includes an aspect pertaining to conditional iterative regrouping of clients depending on the outcome of the evaluation process.

For instance, the method of FIG. 6 can be executed by a system including a central server and multiple clients, e.g., by the system 90 of FIG. 1. Distributed processing at multiple devices is possible. For instance, processors at multiple devices can load program code from respective memories and execute the program code, to participate to the method of FIG. 6 (cf. FIG. 2: device 80).

At box 3104, an initial grouping is performed. This initial grouping can be based on one or more of the following decision criteria: an overall count of groups; number of clients per group; number of pairs of input data - output data in the training datasets, i.e., sizes of the training datasets. This has already been explained above in connection with FIG. 5.

At box 3015, the third training states of the AI model are obtained for each group in accordance with the initial grouping of box 3104. For instance, if there are *M* groups, there are *M* third training states.

Then, at box 3110, a current group from all current groups is selected. In particular, a current group is selected from a set of all groups that have not yet been previously evaluated.

At optional box 3115, it can be determined whether the currently selected group of the current iteration of box 3110 is trusted. In a real-world setting, the trust associated to certain clients would be higher than the remaining clients. The trust level may be assigned by the central authority. This means that the trust-worthy clients generate cleaner data or less noisy data when compared to others. If the model to evaluated arises from a group consisting of trust-worthy clients, then the respective evaluation may be skipped because this group does not yield bad/noisy data. According to examples, evaluating the third training state of the AI model for a trusted group (i.e., a group comprising only or a majority of trusted clients) can be skipped/bypassed, as indicated by the "yes"-path exiting box 3115.

At box 3120, the third training state of the AI model of the currently selected group in the current iteration of box 3110 is evaluated against a current benchmark. Typically, the current benchmark is the AI model previously deployed by the central authority, i.e., in the terminology used above the AI model in the first training state.

Box 3120 can include a cross-evaluation process. For this, the AI model in the third training state associated with the currently selected group can be provided to one or more clients of one or more remaining groups and locally evaluated against the current benchmark based on their respective local training datasets. In some examples, the respective third training state can be provided to all other clients of all remaining groups for the evaluation against their local training datasets.

At box 3125, is determined whether the respective third training state of the AI model of the currently selected group (current iteration of box 3110) meets a predefined benchmark, i.e., the outcome of box 3120 is judged. For instance, it can be determined whether the respective third training state of the AI model performs better than the first training state of the AI model for all remaining clients.

If the predefined benchmark is met, the third training state currently assessed is added to a combination queue box 3145. Also, the respective group is labeled as evaluated so that it will not be processed in any further iteration of box 3110. Subsequently, at box 3150, it is determined whether a further group that has not yet been evaluated is to be processed at a further iteration of box 3110.

If, however, at box 3125 the performance of the respective third training state of the AI model does not meet the predefined benchmark, then, box 3130 is executed. At box 3130 it is determined whether the currently selected group (current iteration of box 3110) includes more than a lower threshold of clients (e.g., configured by the central authority). For instance, it could be checked whether the currently selected group includes more than a single client. In the affirmative, box 3135 is executed. If the check at box 3110 is not passed, the current group is discarded, i.e., labeled as evaluated and since it has a negative evaluation result it is not added the combination queue. Else, it is judged that the current group can be further broken down into smaller groups so that at box 3135, responsive to said evaluating of the AI in the current third training state not meeting a predefined benchmark, the clients previously associated with the current group are associated with multiple newly-formed groups that replace the respective group. Further, the weights of the AI model in the second training states for all clients in each of the newly-formed groups are combined; to obtain multiple new third training states. Then, box 3110 is repeated.

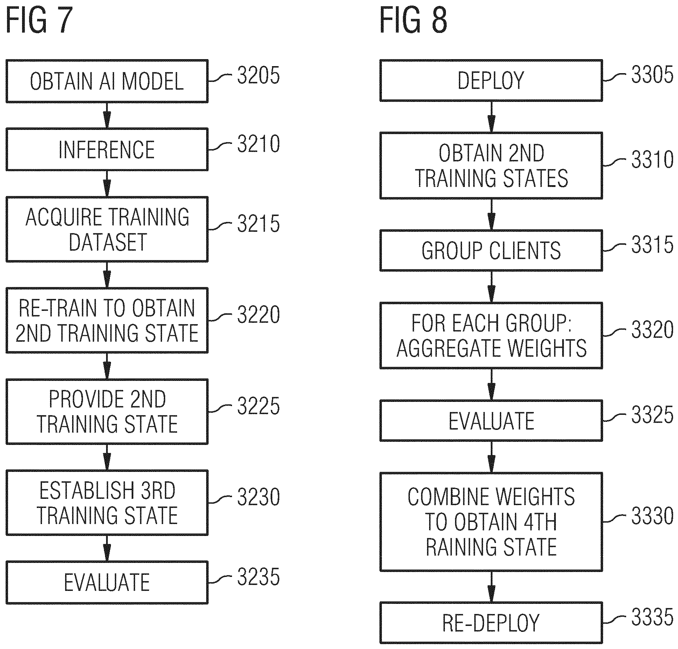

FIG. 7 is a flowchart of a method according to various examples. The method of FIG. 7 can be executed by one of multiple clients, e.g., by one of the clients 92, 93, 94 as illustrated in FIG. 1. For instance, a processor can load program code from a respective memory and execute the program code, to execute the method of FIG. 7 (cf. FIG. 2: processor 97 and memory 99).

The method of FIG. 7 implements client-side processing of the methods of FIG. 5 and optionally FIG. 6.

At box 3205, an AI model in a first training state is obtained. I.e., the client is being deployed with the AI model in the first training state. The AI model in the first training state can be obtained from a central authority.

Respective techniques with respect to deploying an AI model had been previously discussed in connection with box 3005 of FIG. 5.

At box 3210, the AI model in the first training state as obtained in box 3205 is used for performing inference; this has been previously explained in connection with box 3010 in FIG. 5.

At box 3215, a training dataset is acquired, e.g., based on the performing of the inference at box 3210. Respective techniques have been previously discussed in connection with box 3020 of FIG. 5.

At box 3220, re-training is performed, to yield a second training state of the AI model. Respective techniques have been previously discussed in connection with FIG. 5: box 3020; as well as in connection with FIG. 3.

At box 3225, the AI model in the second training state, i.e., the weights thereof, are provided to, e.g., a central authority or one more further clients. The training dataset can be locally retained at the client, to maintain privacy.

Then, at box 3230, the AI model in multiple third training states is established based on information obtained from a least one of the central authority or one more further clients. For instance, it would be possible to obtain data defining the multiple third training states from the central authority or other clients.

As previously explained in connection with box 3040, a third training state is obtained by combining weights of multiple second training states. This combination can be executed at the central authority or other clients. It would also be possible to execute such combination at the client executing the method of FIG. 7; in which case box 3230 can include obtaining the AI model in multiple second training states from multiple further clients or the central authority and associating the client and the multiple further clients with multiple groups and then aggregate the weights of the AI model in the second training states associated with the client in each group to obtain a respective third training state of the AI model. Here, it would be possible to take into account grouping information obtained from the central authority, e.g., indicative of a mapping of clients to groups or one or more grouping criteria, e.g., size of training datasets, etc..

At box 3235, the third training states are evaluated. Respective techniques have been previously discussed in connection with box 3045.

FIG. 8 is a flowchart of a method according to various examples. FIG. 8 pertains to server-side processing, i.e., logic implemented at a central authority, associated with the method of FIG. 5 and optionally with the method of FIG. 6. For instance, a processor at a server can load program code from respective memory and execute the program code, to execute the method of FIG. 8 (cf. FIG. 2: processor 97 and memory 99).

At box 3305, the AI model in the first training state is deployed to multiple clients. Respective techniques have been previously discussed in connection with box 3005 of FIG. 5.

At box 3310, the AI model in respective second training states are obtained from each of multiple clients. Box 3310 is interrelated to box 3225 discussed in connection with FIG. 7 above.

At box 3315, the multiple clients are associated with multiple groups. Respective techniques have been discussed previously in connection with box 3035 of the method of FIG. 5.

At box 3320, for each of the multiple groups, the weights of the AI model in the second training states associated with clients within the respective group are aggregated. Thereby, the AI model in multiple third training states is obtained.

At box 3325, the multiple third training states are evaluated. This can be either implemented at the server, by benchmarking against a local training dataset and comparing, e.g., to the performance of the AI model in the first training status deployed in box 3305; or can be offloaded to the multiple clients by providing the AI model in the third training states to multiple clients.

At box 3330, weights of the AI model in the second training state and/or third training states are aggregated based on the evaluation result of box 3325, to obtain the AI model in the fourth training state. Respective techniques have been previously discussed in connection with box 3050, as well as in connection with FIG. 6, in particular the performance check at box 3125 and following boxes.

It is then possible to re-deploy the AI model in the fourth training state at box 3335.

While FIG. 8 illustrates a scenario in which the grouping of the clients is executed at the central authority at box 3315 and the weights of the AI model in the second training states are also aggregated at the central authority at box 3320, in some scenarios - as previously discussed in connection with FIG. 7: box 3230 - it would also be possible that such aggregation and/or grouping is executed at the clients. For this, assistive grouping information can be provided by the central authority to the multiple clients.

Summarizing, techniques have been disclosed for re-retraining and evaluating an AI model. The disclosed evaluation process is faster than conventional evaluation techniques as it requires fewer operations. Distributed evaluation on real world training datasets is possible. Furthermore, the evaluation mechanism aids in isolating clients that would lower the performance of the overall algorithm if taken into consideration.

Further summarizing, at least the following EXAMPLES have been disclosed.

EXAMPLE 1. A computer-implemented method, comprising:

- deploying (3005) an artificial intelligence model in a first training state (81) to multiple clients (80, 92, 92-1, 92-2, 92-3, 93, 94);
- at each of the multiple clients (80, 92, 92-1, 92-2, 92-3, 93, 94): acquiring (3015) a respective training dataset (82) ;
- at each of the multiple clients (80, 92, 92-1, 92-2, 92-3, 93, 94): re-training (3020) the artificial intelligence model to obtain the artificial intelligence model in a respective second training state (83, 83-1, 83-2, 83-3);
- associating (3035) the multiple clients (80, 92, 92-1, 92-2, 92-3, 93, 94) with multiple groups (70);
- for each of the multiple groups (70): aggregating (3040) weights of the artificial intelligence model in the second training state (83, 83-1, 83-2, 83-3) associated with clients (80, 92, 92-1, 92-2, 92-3, 93, 94) within the respective group (70), to obtain the artificial intelligence model in a respective third training state (85); and
- evaluating (3045) the artificial intelligence model in each of the third training states (85).

EXAMPLE 2. The computer-implemented method of EXAMPLE 1,
wherein the artificial intelligence model in each of the third training states (85) is evaluated using a cross-evaluation process at the multiple clients (80, 92, 92-1, 92-2, 92-3, 93, 94).

EXAMPLE 3. The computer-implemented method of EXAMPLE 2,
wherein the cross-evaluation process comprises a benchmark against performance of the artificial intelligence model in the first training state (81), the benchmark being based on the respective training dataset at the respective client (80, 92, 92-1, 92-2, 92-3, 93, 94).

EXAMPLE 4. The computer-implemented method of EXAMPLE 2 or 3,
wherein the cross-evaluation process includes providing the artificial intelligence model in the respective third training state (85) associated with a given one of the multiple groups (70) to at least one further group (70) of the multiple groups (70) and benchmarking the artificial intelligence model in the respective third training state (85) associated with the given one of the multiple groups at each of the at least one further group.

EXAMPLE 5. The computer-implemented method of any one of the preceding EXAMPLEs, further comprising:

- for each of the multiple groups: responsive to said evaluating of the artificial intelligence model in the respective third training state not meeting a predefined benchmark, re-associating the clients previously associated with the respective group with multiple newly-formed groups replacing the respective group and repeating said aggregating of the weights and evaluating for the newly formed groups.

EXAMPLE 6. The computer-implemented method of any one of the preceding EXAMPLEs, further comprising:

- at each one of the multiple clients (80, 92, 92-1, 92-2, 92-3, 93, 94): responsive to identifying at least one of the respective training data sets or the artificial intelligence model in the respective second training state (83, 83-1, 83-2, 83-3) as an outlier, suppressing an impact of the artificial intelligence model in the respective second training state (83, 83-1, 83-2, 83-3) onto said aggregating of the weights.

EXAMPLE 7. The computer-implemented method of any one of the preceding EXAMPLEs, further comprising:

- bypassing (3115) said evaluating for at least one group (70) comprising clients (80, 92, 92-1, 92-2, 92-3, 93, 94) labeled as trustworthy.

EXAMPLE 8. The computer-implemented method of any one of the preceding EXAMPLEs, wherein the groups (70) are non-overlapping.
EXAMPLE 9. The computer-implemented method of any one of the preceding EXAMPLEs, wherein the clients (80, 92, 92-1, 92-2, 92-3, 93, 94) are associated with the groups (70) depending on one or more predefined criteria, the one or more predefined criteria being selected from: an overall count of the groups (70); a number of clients per group (70); a size of the training datasets at each of the multiple clients (80, 92, 92-1, 92-2, 92-3, 93, 94).
EXAMPLE 10. The computer-implemented method of any one of the preceding EXAMPLEs, further comprising:

- upon said evaluating of the artificial intelligence model in each of the third training states (85): aggregating (3050) weights of the artificial intelligence model in those third training states (85) associated with a positive result of said evaluating, to obtain the artificial intelligence model in a fourth training state, and deploying (3005) the artificial intelligence model in the fourth training state.

EXAMPLE 11. The computer-implemented method of any one of the preceding EXAMPLEs, wherein the artificial intelligence model performs one or more medical imaging processing tasks.
EXAMPLE 12. A computer-implemented method for use in a client, comprising:

- obtaining (3005), from a central authority, an artificial intelligence model in a first training state;
- performing (3010) inference based on the artificial intelligence model in the first training state;
- acquiring (3015) a training dataset based on said performing of the inference;
- re-training (3020) the artificial intelligence model based on the training dataset, to obtain the artificial intelligence model in a second training state;
- providing the artificial intelligence model in the second training state to at least one of the central authority and one or more further clients;
- establishing the artificial intelligence model in multiple third training states based on information obtained from at least one of the central authority or the one or more further clients;
- evaluating the artificial intelligence model in each of the multiple third training states based on benchmarking against performance of the artificial intelligence model in the third training state and based on the training dataset.

EXAMPLE 13. The computer-implemented method of EXAMPLE 12, wherein said establishing of the artificial intelligence model in the multiple third training states comprises:

- obtaining the artificial intelligence model in the multiple third training states from the central authority.

EXAMPLE 14. The computer-implemented method of EXAMPLE 12, wherein said establishing of the artificial intelligence model in the multiple third training states comprises:

- obtaining the artificial intelligence model in multiple second training states from the multiple further clients,
- associating the client and the multiple further clients with multiple groups,
- for each of the multiple groups: aggregating weights of the artificial intelligence model in the second training state associated with clients within the respective group, to obtain the artificial intelligence model in the respective third training state.

EXAMPLE 15. The computer-implemented method of EXAMPLE 14, wherein the client and the multiple further clients are associated with the multiple groups based on grouping information obtained from the central authority.
EXAMPLE 16. A computer-implemented method for use in a central authority, the method comprising:

- deploying an artificial intelligence model in a first training state to multiple clients;
- obtaining the artificial intelligence model in respective second training states from each of the multiple clients;

- associating the multiple clients with multiple groups;
- for each of the multiple groups: aggregating weights of the artificial intelligence model in the second training states associated with clients within the respective group, to obtain the artificial intelligence model in a respective third training state; and
- providing the artificial intelligence model in the third training states to the multiple clients for evaluation.

EXAMPLE 17. The computer-implemented method of EXAMPLE 16, further comprising:

- obtaining an evaluation results from the multiple clients,
- aggregating weights of the artificial intelligence model in the second training states and/or third training states based on the evaluation results, to obtain the artificial intelligence model in a fourth training state, and
- deploying the artificial intelligence model in the fourth training state to the multiple clients.

EXAMPLE 18. A computer-implemented method for use in a central authority, the method comprising:

- deploying an artificial intelligence model in a first training state to multiple clients,
- providing grouping information to the multiple clients, the grouping information being indicative of association of the multiple clients with multiple groups for aggregation of weights of the artificial intelligence model in respective second training states in a cross-evaluation process at the multiple clients.

EXAMPLE 19. A system, comprising a central authority and multiple clients, wherein the system is configured to execute the method of EXAMPLE 1.

EXAMPLE 20. A client comprising a processor and a memory, the processor being configured to load program code from the memory and to execute the program code, wherein execution of the program code causes the processor to perform the method of 12.

EXAMPLE 21. A central server configured to implement a central authority for multiple clients, the central server comprising a processor and a memory, the processor being configured to load program code from the memory and to execute the program code, wherein execution of the program code causes the processor to perform the method of EXAMPLE 16 or EXAMPLE 18.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

**Claims**

1. A computer-implemented method, comprising:

   - deploying (3005) an artificial intelligence model in a first training state (81) to multiple clients (80, 92, 92-1, 92-2, 92-3, 93, 94);
   - at each of the multiple clients (80, 92, 92-1, 92-2, 92-3, 93, 94): acquiring (3015) a respective training dataset (82) ;
   - at each of the multiple clients (80, 92, 92-1, 92-2, 92-3, 93, 94): re-training (3020) the artificial intelligence model to obtain the artificial intelligence model in a respective second training state (83, 83-1, 83-2, 83-3);
   - associating (3035) the multiple clients (80, 92, 92-1, 92-2, 92-3, 93, 94) with multiple groups (70);
   - for each of the multiple groups (70): aggregating (3040) weights of the artificial intelligence model in the second training state (83, 83-1, 83-2, 83-3) associated with clients (80, 92, 92-1, 92-2, 92-3, 93, 94) within the respective group (70), to obtain the artificial intelligence model in a respective third training state (85); and
   - evaluating (3045) the artificial intelligence model in each of the third training states (85).

2. The computer-implemented method of claim 1, wherein the artificial intelligence model in each of the third training states (85) is evaluated using a cross-evaluation process at the multiple clients (80, 92, 92-1, 92-2, 92-3, 93, 94).

3. The computer-implemented method of claim 2, wherein the cross-evaluation process comprises a benchmark against performance of the artificial intelligence model in the first training state (81), the benchmark being based on the respective training dataset at the respective client (80, 92, 92-1, 92-2, 92-3, 93, 94).

4. The computer-implemented method of claim 2 or 3, wherein the cross-evaluation process includes providing the artificial intelligence model in the respective third training state (85) associated with a given one of the multiple groups (70) to at least one further group (70) of the multiple groups (70) and benchmarking the artificial intelligence model in the respective third training state (85) associated with the given one of the multiple groups at each of the at least one further group.

5. The computer-implemented method of any one of the preceding claims, further comprising:

   - for each of the multiple groups: responsive to said evaluating of the artificial intelligence model in the respective third training state not meeting a predefined benchmark, re-associating the clients previously associated with the respective group with multiple newly-formed groups replacing the respective group and repeating said aggregating of the weights and evaluating for the newly formed groups.

6. The computer-implemented method of any one of the preceding claims, further comprising:

    - at each one of the multiple clients (80, 92, 92-1, 92-2, 92-3, 93, 94): responsive to identifying at least one of the respective training data sets or the artificial intelligence model in the respective second training state (83, 83-1, 83-2, 83-3) as an outlier, suppressing an impact of the artificial intelligence model in the respective second training state (83, 83-1, 83-2, 83-3) onto said aggregating of the weights.

7. The computer-implemented method of any one of the preceding claims, further comprising:

    - bypassing (3115) said evaluating for at least one group (70) comprising clients (80, 92, 92-1, 92-2, 92-3, 93, 94) labeled as trustworthy.

8. The computer-implemented method of any one of the preceding claims,
wherein the groups (70) are non-overlapping.

9. The computer-implemented method of any one of the preceding claims,
wherein the clients (80, 92, 92-1, 92-2, 92-3, 93, 94) are associated with the groups (70) depending on one or more predefined criteria, the one or more predefined criteria being selected from: an overall count of the groups (70); a number of clients per group (70); a size of the training datasets at each of the multiple clients (80, 92, 92-1, 92-2, 92-3, 93, 94).

10. The computer-implemented method of any one of the preceding claims, further comprising:

    - upon said evaluating of the artificial intelligence model in each of the third training states (85): aggregating (3050) weights of the artificial intelligence model in those third training states (85) associated with a positive result of said evaluating, to obtain the artificial intelligence model in a fourth training state, and deploying (3005) the artificial intelligence model in the fourth training state.

11. The computer-implemented method of any one of the preceding claims,

wherein the artificial intelligence model performs one or more medical imaging processing tasks.

12. A computer-implemented method for use in a client, comprising:

    - obtaining (3005), from a central authority, an artificial intelligence model in a first training state;
    - performing (3010) inference based on the artificial intelligence model in the first training state;
    - acquiring (3015) a training dataset based on said performing of the inference;
    - re-training (3020) the artificial intelligence model based on the training dataset, to obtain the artificial intelligence model in a second training state;
    - providing the artificial intelligence model in the second training state to at least one of the central authority and one or more further clients;
    - establishing the artificial intelligence model in multiple third training states based on information obtained from at least one of the central authority or the one or more further clients;
    - evaluating the artificial intelligence model in each of the multiple third training states based on benchmarking against performance of the artificial intelligence model in the third training state and based on the training dataset.

13. The computer-implemented method of claim 12, wherein said establishing of the artificial intelligence model in the multiple third training states comprises:

    - obtaining the artificial intelligence model in the multiple third training states from the central authority.

14. The computer-implemented method of claim 12, wherein said establishing of the artificial intelligence model in the multiple third training states comprises:

    - obtaining the artificial intelligence model in multiple second training states from the multiple further clients,
    - associating the client and the multiple further clients with multiple groups,
    - for each of the multiple groups: aggregating weights of the artificial intelligence model in the second training state associated with clients within the respective group, to obtain the artificial intelligence model in the respective third training state.

15. The computer-implemented method of claim 14, wherein the client and the multiple further clients are associated with the multiple groups based on grouping information obtained from the central authority.

## FIG 1

SERVER — 91

90

92

CLIENT

95

94

CLIENT

CLIENT — 93

## FIG 2

80

HMI — 96

99

CPU — 97

MEM

IF — 98

## FIG 3

92, 93, 94

the artificial intelligence model in the first training state — 81

a respective training dataset — 82

the artificial intelligence model in the second training state — 83

# FIG 4

70

92-1 92-2 92-3

CLIENT  CLIENT  ···  CLIENT

the artificial intelligence model in the third training state — 85

# FIG 5

DEPLOY — 3005

AT EACH CLIENT: INFERENCE — 3010

3091

AT EACH CLIENT: ACQUIRE TRAINING DATSET — 3015

FOR EACH TRAINING DATASET: RE-TRAIN — 3020

3092

OUTLIER REMOVAL — 3025

GROUP CLIENTS — 3035

AT EACH GROUP: COMBINE WEIGHTS — 3040

EVALUATE — 3045

3093

3050
COMBINE WEIGHTS FOR ALL POSITIVELY EVALUATED GROUPS

FIG 6

3093

```
         ┌──────────────────────┐
         │   INITIAL GROUPING   │── 3104
         └──────────────────────┘
                    │
         ┌──────────────────────┐
         │    OBTAIN AI MODEL   │
         │   IN THIRD TRAINING  │── 3105
         │ STATE FOR EACH GROUP │
         └──────────────────────┘
                    │
         ┌──────────────────────┐
    ┌───▶│    SELECT CURRENT    │── 3110 ◀─────────────────┐
    │    │        GROUP         │                          │
    │    └──────────────────────┘                          │
    │               │                                      │
    │             ╱   ╲  3115                               │
    │       Y   ╱TRUSTED?╲                                  │
    │   ┌─────◀           ╲                                 │
    │   │       ╲        ╱                                  │
    │   │         ╲    ╱                                    │
    │   │           │ N                                     │
    │   │  ┌──────────────────────┐                         │
    │   │  │   EVALUATE AGAINST   │                         │
    │   │  │       CURRENT        │── 3120                   │
    │   │  │     BENCHMARK        │                         │
    │   │  └──────────────────────┘                         │
    │   │             │                                     │
    │   │           ╱   ╲ 3125        ╱   ╲ 3130    ┌──────────────────┐
    │   │    PER-  ╱      ╲      N   ╱       ╲  Y   │ BREAK DOWN INTO  │
    │   │  FORMANCE         ╲──────╱ ASSOCIATED╲───▶│MULTIPLE NEW      │── 3135
    │   │       ╲  OK?    ╱         ╲WITH MULTIPLE╱ │GROUPS AND        │
    │   │         ╲      ╱           ╲ CLIENTS? ╱   │COMBINE WEIGHTS   │
    │   │  3145     ╲  ╱ Y             ╲      ╱     └──────────────────┘
    │   │  ┌──────────────────────┐     │ N
    │   └─▶│      ADD TO          │   ┌──────────┐
    │      │    COMBINATION       │   │ DISCARD  │── 3140
    │      │       QUEUE          │   └──────────┘
    │      └──────────────────────┘        │
    │                 │                    │
    │               ╱   ╲ 3150             │
    │         Y   ╱       ╲                │
    └────────────╱ FURTHER  ╲◀─────────────┘
                 ╲  GROUP?  ╱
                  ╲       ╱
                    ╲   ╱
                     │ N
                     ▼
```

# FIG 7

| | |
|---|---|
| OBTAIN AI MODEL | 3205 |
| INFERENCE | 3210 |
| ACQUIRE TRAINING DATASET | 3215 |
| RE-TRAIN TO OBTAIN 2ND TRAINING STATE | 3220 |
| PROVIDE 2ND TRAINING STATE | 3225 |
| ESTABLISH 3RD TRAINING STATE | 3230 |
| EVALUATE | 3235 |

# FIG 8

| | |
|---|---|
| DEPLOY | 3305 |
| OBTAIN 2ND TRAINING STATES | 3310 |
| GROUP CLIENTS | 3315 |
| FOR EACH GROUP: AGGREGATE WEIGHTS | 3320 |
| EVALUATE | 3325 |
| COMBINE WEIGHTS TO OBTAIN 4TH RAINING STATE | 3330 |
| RE-DEPLOY | 3335 |

**Europäisches Patentamt**
**European Patent Office**
**Office européen des brevets**

# EUROPEAN SEARCH REPORT

| Application Number |
| --- |
| EP 23 20 9774 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| X | WO 2023/036184 A1 (HUAWEI CLOUD COMPUTING TECH CO LTD [CN]) 16 March 2023 (2023-03-16) * paragraphs [0009], [0040], [0057], [0058], [0064], [0065], [0087], [0092]; figure 6 * | 1-15 | INV. G06N20/00 |
| X | US 2023/177812 A1 (LI XIAOMENG [CN]) 8 June 2023 (2023-06-08) * paragraphs [0006] - [0009], [0027] * | 1-15 | |

**TECHNICAL FIELDS SEARCHED (IPC)**

G06N

The present search report has been drawn up for all claims

| Place of search | Date of completion of the search | Examiner |
| --- | --- | --- |
| The Hague | 29 April 2024 | Bykowski, Artur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 9774

This annex lists the patent family members relating to the patent documents cited in  the above-mentioned European search report.
The members are as contained in the European Patent Office EDP file on
The European Patent Office is in no way liable for these particulars which are merely given for the purpose of information.

29-04-2024

| Patent document cited in search report | Publication date | Patent family member(s) | Publication date |
|---|---|---|---|
| WO 2023036184   A1 | 16-03-2023 | NONE | |
| US 2023177812   A1 | 08-06-2023 | CN     116244591 A<br>US     2023177812 A1 | 09-06-2023<br>08-06-2023 |

EPO FORM P0459

For more details about this annex : see Official Journal of the European Patent Office, No. 12/82